# EUROPEAN PATENT APPLICATION

(11) **EP 1 897 562 A1**
(43) Date of publication of application: **12.03.2008**
(21) Application number: 06090165.9
(22) Date of filing: 08.09.2006
(51) Int. Cl.: A61K 51/08, A61K 51/04, C12N 15/11, A61P 35/00

(54) **Aptamers labelled with Gallium-68**

(71) Applicant: Bayer Schering Pharma Aktiengesellschaft, 13353 Berlin (DE)
(72) Inventor: Friebe, Matthias, Dr., 13509 Berlin (DE); Hecht, Maren, 12203 Berlin (DE); Noll, Bernhard, Dr., 01705 Freital (DE); Dinkelborg, Ludger, Dr., 13465 Berlin (DE); Lehmann, Lutz, Dr., 13357 Berlin (DE)

(57) **Abstract**

This invention relates to novel nucleotide-based compounds, methods of making radiolabeled compounds and use of such compounds for diagnostic imaging. Provided are compounds according to Formula (I) A - B - L - C , wherein A stands for an aptamer, B is absent or stands for a bridging structure, L is a linker, and C is a metal ion chelator. All substituents are defined in detail in the description. Preferred embodiments of said compounds bind to Tenascin-C.

## Description

### FIELD OF THE INVENTION

This invention relates to novel nucleotide-based compounds, methods of making radiolabeled compounds and use of such compounds for diagnostic imaging.

### BACKGROUND OF THE INVENTION

### Tenascin-C as a potential target for diagnostic imaging in oncology

Besides other interesting targets, tenascin-C, a hexameric glycoprotein that is located primarily in the extracellular matrix (ECM) could serve as an appropriate target for specific molecular imaging of cancerous lesions. The protein is produced by epithelial cells and surrounding mesenchymal cells (see Harold P. Erickson, Annu. Rev. Cell Biol. 5 (1989) 71-92; R Chiquet-Ehrismann, EJ Mackie, CA Pearson, T Sakakura, Tenascin: an Extracellular Matrix Protein Involved in Tissue Interactions during Fetal Development and Oncogenesis, Cell 47 (1986) 131-139; R Chiquet-Ehrismann, P Kalla, CA Pearson, K Beck, M Chiquet, Tenascin Interferes with Fibronectin Action, Cell 53 (1988) 383-390). It is involved in cell proliferation, cancer invasion, metastasis and secondary tumor formation. It is also strongly present in embryonic development and in wound healing but its expression is only very limited in adult normal tissue (see e.g. Bourdon M.A., Wikstrand C.J., Furthermayr H., Metthews T.J., Bigner D.D., Cancer Res. 43 (1983) 2796-2805; N Hiraiwa, H Kida, T Sakakura, M Kusakabe, Induction of tenascin in cancer cells by interactions with embryonic mesenchyme mediated by a diffusible factor, Journal of Cell Science 104 (1993) 289-296).
Tenascin-C is secreted by fibroblasts and glial cells in tissue culture. One of the experimental sources of human tenascin-C is the glioma cell line U251 MG, but also other cells such as U373, PC3 and F9 express large amounts of the protein. Each human tenascin-C subunit includes three types of structural modules: 14 1/2 epidermal growth factor-like repeats, 15 fibronectin (FN)type, three homology repeats, and a COOH-terminal knob which consists of 215 amino acids with homology to the globular domain of the β and γ chains of human fibrinogen.

Due to its high expression in a wide variety of tumors, the protein tenascin-C (TN-C) is an interesting target for multi tumor diagnosis and therapy (see AM Tókés, E Hortoványi, J Kulka, M Jäkel, T Kerény, A Kádár, Tenascin expression and angiogenesis in breast cancer, Pathol. Res. Pract. 195 (1999) 821-828; Y Soini, P Paakko, Tenascin immunoreactivity in lung tumors, Am. J. Clin. Path. 100 (1993) 145-150; S Riedl, A Faissner, P Schlag, A Aon-Herbay, K Koretz, P Möller, altered content and distribution of tenascin in colitis, colon adenoma and colorectal carcinoma, Gastroenterology 103 (1992) 400-406; MM Bonsanto, Klinische und funktionelle Aspekte der Tenascin-Expression im Zuge der Gliomprogression, http://www.ub.uniheidelberg.de/archiv/4305). An antibody against TN-C has been generated and radiolabeled for testing its applicability in radiotherapy (see e.g. G Akabani, DA Reardon, RE Coleman, TZ Wong, SD Metzler, JE Bowsher, DP Barboriak, JM Provenzale, KL Greer, D DeJong, HS Friedman, AH Friedman, XG Zhao, CN Pegram, RE McLendon, DD Bigner, MR Zalutsky, Dosimetry and Radiographic Analysis of 131I-Labeled Anti-Tenascin 81C6 Murine Monoclonal Antibody in Newly Diagnosed Patients with malignant Gliomas: A Phase II Study, The Journal of Nuclear Medicine 46 (2005) 1042-1050; G Paganelli, C Grana, M chinol, M Cremonesi, C De Cicco, F De Braud, C Robertson, S Zurrida, C Casadio, S Zoboli, AG Siccardi, U Veronesi, Antibody-guided three-step therapy for high grade glioma with yttrium-90, European Journal of Nuclear Medicine 26 (1999) 348-357; F Petronzelli, A Pelliccia, AM Anastasi, V D'Alessio, C Albertoni, A Rosi, B Leoni, C De Angelis, G Paganelli, G Palombo, M Dani, P Carminati, R De Santis, Improved Tumor Targeting by Combined Use of Two Antitenascin Antibodies, Clin Cancer Res 11 (2005) 7137s-7145s; MR Zalutsky, RP Moseley, HB Coakham, RE Coleman, DD Bigner, Pharmacokinetics and Tumor Localization of 131I-Labeled Anti-Tenascin Monoclonal Antibody 81 C6 in Patients with Gliomas and Other Intracranial Malignancies, Cancer Research 49 (1989) 2807-2813; G Akabani, I Cokgor, RE Coleman, DG Trotter, TZ Wong, HS Friedman, AH Friedman, A Garcia-Turner, JE Herndon II, D DeLong, RE McLendon, XG Zhao, CN Pegram, JM Provenzale, DD binger, MR Zalutsky, Dosimetry and Dose-Response Relationships in Newly Diagnosed Patients with Malignant Gliomas Treated with lodine-131-Labeled Anti-Tenascin Monoclonal Antibody 81 C6 Therapy, Int. J. Radiation Oncology Biol. Phys. 46 (2000) 947- 958). A high tumor uptake of the radiolabeled antibody has been displayed in these studies. Despite the high affinity binding, this approach is fraught by the large size of antibodies which leads to a long blood-retention time and a slow tissue penetration. This in turn causes an unfavorable tumor-to-non-tumor distribution of the radiolabeled antibody. Furthermore, antibodies can be immunogenic and have high costs of production, two factors that limit their use in diagnostic applications. Synthetic oligonucleotides such as aptamers could overcome these problems. Aptamers are non-immunogenic and usually have a very selective binding to their target. There are hardly any interactions with other tissue which together with a mid-size molecular weight (7-20 kDa) leads to a fast body clearance. A potential binding site for aptamers could be the terminal knob of tenascin-C. Therefore, six possible binding sites would exist per tenascin-C molecule.

### Aptamers

Aptamers are synthetic DNA or RNA molecules that can be selected from oligonucleotide libraries based on their ability to bind a target molecule (M Famulok, G Mayer, Aptamers as Tools in Molecular Biology and Immunology, In: Combinatorial Chemistry in Biology, Current Topics in Microbiology and Immunology (M Famulok, CH Wong, EL Winnacker, Eds.), Springer Verlag Heidelberg, 1999, Vol. 243, 123-136). They fold into three dimensional structures by Watson-Crick base pairing. This three dimensional structure enables aptamers for molecular recognition and can lead to a high affinity and selectivity for a particular target. Their preshoped structures can minimize the entropic loss during binding to the target. Of high interest is their ability to bind to proteins with high specificity (L Cerchia, J Hamm, D Libri, B Tavitian, V de Franciscis, Nucleic acid aptamers in cancer medicine, FEBS Letters 528 (2002), 12-16). Binding affinities are usually in the sub-nanomolar range. It is noteworthy, that aptamers can selectively recognize minor sequence variations of biomolecules and even enantiomers of small molecules with usually several orders of magnitude (SM Nimjee, CP Rusconi, BA Sullenger, Aptamers: An Emerging Class of Therapeutics, Annu. Rev. Med. 56 (2005) 555-583; RR White, BA Sullenger, CP Rusconi, Developing aptamers into therapeutics, The Journal of Clinical Investigation 106 (2000) 929-934).
Aptamers binding to a variety of targets are described in the literature. Among those are aptamers with a high affinity for α-thrombin (MF Kubik, AW Stephens, D Schneider, RA Marlar, D Tasset, High-affinity RNA ligands to human α-thrombin, Nucleic Acids Research 22 (1994) 2619-2626) , HIV-1 reverse transcriptase (C Tuerk, S MacDougal, L Gold, RNA pseudoknots that inhibit human immunodeficiency virus type 1 reverse transcriptase, Proc. Natl. Acad. Sci. U.S.A. 89 (1992) 6988-6992), and basic fibroblast growth factor (D Jellinek, CK Lynott, DB Rifkin, N Janjic, High-affinity RNA ligands to basic fibroblast growth factor inhibit receptor binding, Proc. Natl. Acad. Sci. U.S.A. 90 (1993) 11227-11231). The first aptamer drug that gained approval by the FDA is Macugen, a molecule that was identified by NeXstar (NX-1838) (I Melnikova, Wet age-related macular degeneration, Nature Reviews 4 (2005) 711-712). It specifically targets the vascular endothelial growth factor (VEGF), a protein promoting blood vessel growth, which is a hallmark of neovascular (wet) macular degeneration. Hence, it slows the progress of wet macular degeneration, the leading cause of blindness in people older than 50. Due to their high selectivity for a specific target, aptamers have a high potential as targeting probes in radiodiagnostic imaging and therapy. Their favorable size, which lies in-between that of antibodies and peptides, combined with the high potential binding affinity may allow for high signal to noise ratios.

Such high affinity binding aptamers can be generated by an *in vitro* process called SELEX (Systematic Evolution of Ligands by Exponential enrichment). This iterative process was established by Gold et al. in 1990 (JM Bacher AD Ellington, Nucleic acid selection as a tool for drug discovery, DDT 3 (1998) 265- 273; L Gold, B Polisky, O Uhlenbeck, M Yarus, Diversity of Oligonucleotide Functions, Annu. Rev. Biochem. 64 (1995) 763-797) based on the selection and amplification of the anticipated high affinity binding aptamer. The starting library for the selection of oligonucleotide aptamers contains single stranded DNA or RNA oligonucleotides with a central region of randomized sequences (up to 10¹⁵ different sequences) abutted on constant regions for subsequent reverse transcription and amplification. These single stranded oligonucleotides are incubated with a target molecule (e.g. tenascin-C) and then partitioned by passage through a nitrocellulose filter: Those oligonucleotides bound to the protein are collected and reverse transcribed into RNA (or DNA), and amplified with the polymerase chain reaction (PCR) to create a new pool that can be used for the next round of SELEX. After several repetitions (usually 8-12), typically performed with increasing stringency, the selected ligands are sequenced and evaluated for their binding capabilities.

### TTA1: A Tenascin-C binding aptamer

An aptamer against the human matrix protein tenascin-C has been identified via the SELEX process using a tenascin-C expressing U251 cell line as well as purified human tenascin-C protein and was first published by Hicke et al (BJ Hicke, C Marion, YF Chang, T Gould, CK Lynott, D Parma, PG Schmidt, S Warren, Tenascin-C Aptamers Are Generated Using Tumor Cells and Purified Protein, The Journal of Biological Chemistry 276 (2001) 48644 - 48654; WO 01/09390 A1). Random 2'F stabilized pyrimidine RNA sequences were incubated with the protein expressing cell line. After 9 rounds the best binding sequences were incubated with the purified tenascin-C. Two more rounds were needed to discover a sequence with a binding affinity to tenascin-C of 1 x 10⁻⁹ M. The final aptamer was then modified to be able to withstand *in vivo* degradation. The modifications covered size minimization, further nuclease stabilization and the attachment of a bioconjugation handle. The size minimization included the replacement of 17 bases by a (CH₂O)₆ group without loss in affinity. Nuclease resistance was already introduced before the SELEX process by using 2'F pyrimidines, further stabilization was achieved by converting 2'OH purines into 2'OMe purines. Hicke et al. found that the conversion of 2'OH in five guanodines (G) led to a loss of activity: G¹, G⁹, G¹¹, G¹⁴ and G¹⁷. Hence, these five nucleotides remained 2'OH. In addition a 3'cap was added to further increase the nuclease stability. Finally, a C₆-NH₂ linker was added to the 5' end of the oligonucleotide and the final aptamer was tested again for its binding affinity. The synthetic oligonucleotide has a K_{D} of 5 x 10⁻⁹ M which represents a five fold reduction in affinity from the parent full size aptamer. The final molecule was named TTA1, which stands for Tenascin-C targeting aptamer-1 and the sequence was determined to be:

NH2-C6-5'-GGG AGG ACG-(CH2CH2O)6-CGU CGC CGU AAU GGA UGU UUU GCU CCC UG-
3'-3'-T

In conclusion, TTA1 consists of 39 nucleotides which are stabilized against nuclease degradation by the substitution of the 2'OH group versus a 2'fluoro substituent of the ribose backbone of pyrimidine and versus 2'-O-methyl substituents in the purine nucleic acids. In addition, the 3' end was protected against exonuclease degradation by inverting the 3' nucleotide to form a new 5'OH, with a 3'-3' linkage to a penultimate base. The amine group on the linker can be used for the introduction of e.g. chelating ligands or dyes. TTA1 is characterized by a 13.3 kDa molecular weight and a binding affinity of K_{D} = 5 nM for human tenascin-C.

TTA1 coupled to mercapto acetyl diglycine (MAG₂), an N₃S coordinating ligand, suitable for the subsequent radio labeling with [^{99m}Tc(V)O]³⁺, is the current lead structure. It is assumed that stem 2 and stem 3 are vital for the binding to the target protein while stem 1 is important for the structural stability.
The radiolabeled lead compound was tested *in vitro* and *in vivo.* An organ distribution study in tumor bearing mice revealed a significant tumor uptake of the lead compound along with a considerable uptake of activity in the excretory organs such as liver and kidneys. A slow hepatobilliary clearance concomitant with a significant intestinal uptake usually leads to unfavorable diagnostic imaging due to a rather high background activity.

The object of the present invention is to provide new compounds that have improved properties and are suitable for diagnostic imaging.

### SUMMARY OF THE INVENTION

The present invention provides novel compounds of Formula I and compounds according to Formula I that are labeled with a lanthanide or lanthanide like metal.

The present invention also provides compositions comprising radiolabeled compound of Formula I and a pharmaceutically acceptable carrier or diluent.

The invention further provides a method of imaging diseases, the method comprising introducing into a patient a detectable quantity of a labeled compound of Formula I or pharmaceutically acceptable salt, ester, or amide thereof.

The invention provides the compounds of Formula I or labeled compounds according to Formula I for use as a medicament.

Another aspect of the invention is directed to the use of compounds of Formula I for the manufacture of a medicament.

The present invention also provides a kit for preparing a radiopharmaceutical preparation, said kit comprising a sealed vial containing a predetermined quantity of the compound of Formula I.

Another aspect of the invention is directed to the use of labeled compounds of Formula I for PET imaging.

More specifically the compounds of this invention are useful for the imaging of a variety of cancers including but not limited to: carcinoma such as bladder, breast, colon, kidney, liver, lung, including small cell lung cancer, esophagus, gall-bladder, ovary, pancreas, stomach, cervix, thyroid, prostate, and skin, hematopoetic tumors of lymphoid and myeloid lineage, tumors of mesenchymal origin, tumors of central peripheral nervous systems, other tumors, including melanoma, seminoma, teratocarcinoma, osteosarcoma, xeroderma pigmentosum, keratoxanthoma, thyroid follicular cancer, and Karposi's sarcoma.

### DETAILED DESCRIPTION OF THE INVENTION

In a first aspect, the present invention is directed to compounds according to Formula I

A-B-L-C (I)

wherein,
**A** stands for an aptamer.

For the purpose of this invention, the term "aptamer" refers to synthetic molecules, comprising from 4 to 100 nucleotides, wherein at least two single nucleotides are connected to each other via a phosphodiester linkage. Said aptamers have the ability to bind specifically to a target molecule (see e.g. M Famulok, G Mayer, Aptamers as Tools in Molecular Biology and Immunology, In: Combinatorial Chemistry in Biology, Current Topics in Microbiology and Immunology (M Famulok, CH Wong, EL Winnacker, Eds.), Springer Verlag Heidelberg, 1999, Vol. 243, 123-136). There are many ways known to the skilled person of how to generate such aptamers that have specificity for a certain target molecule. An example is given in WO 01/09390, the disclosure of which is hereby incorporated by reference. Said aptamers may comprise substituted or non-substituted natural and non-natural nucleotides. Aptamers can be synthesized in vitro using e.g. an automated synthesizer (see e.g. S Verma, F Eckstein, Modified Oligonucleotides: Synthesis and Strategy for Users, Annual Reviews of Biochemistry 67 (1998) 99-134). Aptamers according to the present invention can be stabilized against nuclease degradation e.g. by the substitution of the 2'-OH group versus a 2'-fluoro substituent of the ribose backbone of pyrimidine and versus 2'-O-methyl substituents in the purine nucleic acids. In addition, the 3' end of an aptamer can be protected against exonuclease degradation by inverting the 3' nucleotide to form a new 5'-OH group, with a 3' to 3' linkage to a penultimate base (see e.g. BJ Hicke, C Marion, YF Chang, T Gould, CK Lynott, D Parma, PG Schmidt, S Warren, Tenascin-C Aptamers Are Generated Using Tumor Cells and Purified Protein, The Journal of Biological Chemistry 276 (2001) 48644 - 48654)).

For the purpose of this invention, the term "nucleotide" refers to molecules comprising a nitrogen-containing base, a 5-carbon sugar, and one or more phosphate groups. Examples of said base comprise, but are not limited to, adenine, guanine, cytosine, uracil, and thymine. Also non-natural, substituted or non-substituted bases are included. Examples of 5-carbon sugar comprise, but are not limited to, D-ribose, and D-2-desoxyribose. Also other natural and non-natural, substituted or non-substituted 5-carbon sugars are included. Nucleotides as used in this invention may comprise from one to three phosphates.

In preferred embodiments of compounds according to Formula I, aptamers **A** are selected from Tenascin-C binding aptamers.

For the purpose of this invention, the term "Tenascin-C binding" shall have the following meaning: Aptamers that are Tenascin-C binding show a binding affinity towards Tenascin-C of 1 mM or less, wherein less means a more specific affinity in the nanomolar and/or subnanomolar range. This binding affinity is measured using a well established assay, which is easy to perform and well known to the person skilled in the art. This assay is described in detail e.g. in WO 01/09390, which is hereby incorporated by reference.

Further preferred embodiments of said aptamers **A** have a binding affinity towards Tenascin-C of 100nM or less, with a further preferred range of 50nM to 1pM.

In preferred embodiments of compounds according to Formula I, aptamers **A** comprise from 10 to 50 nucleotides.

Further preferred embodiments of said aptamer **A** including the bridging structure **B** are selected from the group of

**TTA1:** 5'-NH2-C6-O-PO2-O-GGG AGG ACG-(CH2CH2O)6-CGU CGC CGU AAU GGA UGU
UUU GCU CCC UG-3'-3'-T ,

**TTA2:** 5'-NH2-C6-O-PO2-O-mGmGmG mAmGG mAfCmG-(CH2CH2O)6-fCGfU fCGfC fCGfU
mAmAfU mGmGmA fUmGfU fUfUfU mGfCfU fCfCfC UmG-3'-3'-T ,

TTA3: 5'-NH2-C6-O-PO2-O-mGmGmG mAmGG mAfCmG-(CH2CH2O)6-fCGfU fCGfC fCGfU
mAmAfU mGmGmA fUmGfU fUfUfU mGfCfU fCfCfC UG-3'-3'-T ,

**TTA4:** 5'-mGmGmG mAmGG mAfCmG-(CH2CH2O)6-fCGfU fCGfC fCGfU mAmAfU mGmGmA
fUmGfU fUfUfU mGfCfU fC(C6-NH2)CfC-3'-3'-T ,

**TTA5:** 5'-(aminomodifier C2 dT)-mA mGmGmG mAmGG mAfCmG-(CH2CH2O)6-fCGfU fCGfC
fCGfU mAmAfU mGmGmA fUmGfU fUfUfU mGfCfU fCfCfC fUmG-3'-3'-T ,

TTA6: 5'-NH2-C6-O-SPO-O-mGmGmG mAmGG mAfCmG-(CH2CH2O)6-fCGfU fCGfC fCGfU
mAmAfU mGmGmA fUmGfU fUfUfU mGfCfU fCfCfC fUmG-3'-3'-T ,

**TTA7:** 5'-LGLGLG LALGmG mAfCrG-(CH2OCH2)6-fCGfU fCGfC fCGfU mAmAfU mGmGmA
fUmGfU fUfUfU mGLCLU LCLCLC-(CH2)6NH2-3', and

**TTA8:** 5'-mGmGmG mAmGmG mAfCrG-(CH2OCH2)6-fCGfU fCGfC fCGfU mAmAfU mGmGmA
fUmGfU fUfUfU mGmCmU mCmCmC-(CH2)6NH2-3'

**B** is absent or stands for a bridging structure.
The 5' end of an aptamer may be substituted at the free phosphate group with a bridging structure that allows for the conjugation of further chemical structures, e.g. the linker L. This bridging structure comprises a substituted or un-substituted lower un-branched or branched alkyl, lower un-branched or branched alkene, or substituted or un-substituted lower un-branched or branched aldehyde carrying an amine functionality. As used in the specification and appended claims, unless specified to the contrary, the term "lower un-branched or branched alkyl" shall have the following meaning: a straight or branched chain monovalent or divalent radical consisting solely of carbon and hydrogen, containing no unsaturation and having from one to eight carbon atoms, e.g. but not limited to methyl, ethyl, n-propyl, n-pentyl, 1,1-dimethylethyl (t-butyl), n-heptyl, and the like. As used in the specification and appended claims, unless specified to the contrary, the term "lower un-branched or branched alkene" shall have the following meaning: a straight or branched chain monovalent or divalent radical consisting solely of carbon and hydrogen, containing at least one unsaturation and having from one to eight carbon atoms. As used in the specification and appended claims, unless specified to the contrary, the term "lower un-branched or branched aldehyde" shall have the following meaning: a straight or branched chain monovalent or divalent radical consisting solely of carbon and hydrogen and at least one aldehyde group and having from one to eight carbon atoms.

In preferred embodiments of compounds according to Formula I, the bridging structure **B** is hexyl amine.

**L** is a linker.
For the purpose of this invention, the term "linker' refers to a chemical entity that connects the aptamer A via an amine group of the bridging structure B or of the aptamer with the chelator C. The linker itself can be a bond or is characterized by the presence of a free mercapto (SH)-functionality that can be used to connect the chelator C to the molecule. Examples of such linker L are, but are not limited to, a bond, substituted or un-substituted lower un-branched or branched alkyl, lower un-branched or branched alkene, or lower un-branched or branched aldehyde carrying at least one mercapto-group, a chemical structure carrying at least one free mercapto-group comprising one amino acid or an amino acid sequence of two to 20 amino acids, a polyethyleneglycol (PEG) sequence or any combinations thereof.

In preferred embodiments of compounds according to Formula I, **L** is a chemical structure carrying at least one free mercapto-group, wherein said chemical structure comprises an amino acid sequence of 2 to 10 amino acids, mercaptoacetyl-glycine-glycine (see e.g. S Hilger, MC Willis, M Wolters, WA Pieken, Tc-99m-labeling of modified RNA, Nucleosides Nucleotides 18 (1999) 1479 - 1481, B Johannsen, H Spies, Technetium(V) Chemistry as Relevant to Nuclear Medicine, Topics in Current Chemistry 176 (1996) 78 - 120), mercaptobutyrimidyl, mercapto-substituted C₁-C₄ alkyl, mercapto-substituted C₁-C₄ alkene, or mercapto-substituted C₁-C₄ aldehyde.

For the purpose of the present invention the term "amino acid sequence" is defined herein as a polyamide obtainable by polycondensation of at least two amino acids. For the purpose of the present invention the term "amino acid" means any molecule comprising at least one amino group and at least one carboxyl group, but no peptide bond within the molecule. In other words, an amino acid is a molecule that has a carboxylic acid functionality and an amine nitrogen having at least one free hydrogen, preferably in alpha position thereto, but no amide bond in the molecule structure. Thus, a dipeptide having a free amino group at the N-terminus and a free carboxyl group at the C-terminus is not to be considered as a single "amino acid" in the above definition.
An amide bond as used herein means any covalent bond having the structure 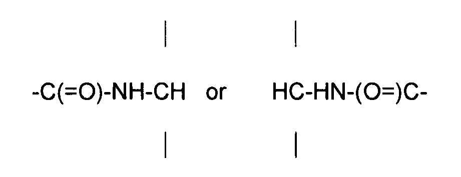 wherein the carbonyl group is provided by one molecule and the NH-group is provided by the other molecule to be joined. The amide bonds between two adjacent amino acid residues which are obtained from such a polycondensation are defined as "peptide bonds". Optionally, the nitrogen atoms of the polyamide backbone (indicated as NH above) may be independently alkylated, e.g. with -C₁-C₆-alkyl, preferably -CH₃.

For the purpose of the specification an amino acid residue is derived from the corresponding amino acid by forming a peptide bond with another amino acid.

For the purpose of the specification an amino acid sequence may comprise naturally occurring and/or artificial amino acid residues, proteinogenic and/or non-proteinogenic amino acid residues. The non-proteinogenic amino acid residues may be further classified as (a) homo analogues of proteinogenic amino acids, (b) β-homo analogues of proteinogenic amino acid residues and (c) further non-proteinogenic amino acid residues.

Accordingly, the amino acid residues are derived from the corresponding amino acids, e.g. from
• proteinogenic amino acids, namely Ala, Arg, Asn, Asp, Cys, Gln, Glu, Gly, His, Ile, Leu, Lys, Met, Phe, Pro, Ser, Thr, Trp, Tyr and Val; or
• non-proteinogenic amino acids, such as
   o homo analogues of proteinogenic amino acids wherein the sidechain has been extended by a methylene group, e.g. Homoalanine (Hal), Homoarginine (Har), Homocysteine (Hcy), Homoglutamine (Hgl), Homohistidine (Hhi), Homoisoleucine (Hil), Homoleucine (Hle), Homolysine (Hly), Homomethionine (Hme), Homophenylalanine (Hph), Homoproline (Hpr), Homoserine (Hse), Homothreonine (Hth), Homotryptophane (Htr), Homotyrosine (Hty) and Homovaline (Hva);
   o β-homo analogues of proteinogenic amino acids wherein a methylene group has been inserted between the α-carbon and the carboxyl group yielding β-amino acids, e.g. β-Homoalanine (βHal), β-Homoarginine (βHar), β-Homoasparagine (βHas), β-Homocysteine (βHcy), β-Homoglutamine (βHgl), β-Homohistidine (βHhi), β-Homoisoleucine (βHil), β-Homoleucine (βHle), β-Homolysine (βHly), β-Homomethionine (βHme), β-Homophenylalanine (βHph), β-Homoproline (βHpr), β-Homoserine (βHse), β-Homothreonine (βHth), β-Homotryptophane (βHtr), β-Homotyrosine (βHty) and β-Homovaline (βHva);
   o further non-proteinogenic amino acids, e.g. α-Aminoadipic acid (Aad), β-Aminoadipic acid (βAad), α-Aminobutyric acid (Abu), α-Aminoisobutyric acid (Aib), β-Alanine (βAla), 4-Aminobutyric acid (4-Abu), 5-Aminovaleric acid (5-Ava), 6-Aminohexanoic acid (6-Ahx), 8-Aminooctanoic acid (8-Aoc), 9-Aminononanoic acid (9-Anc), 10-Aminodecanoic acid (10-Adc), 12-Aminododecanoic acid (12-Ado), α-Aminosuberic acid (Asu), Azetidine-2-carboxylic acid (Aze), β-Cyclohexylalanine (Cha), Citrulline (Cit), Dehydroalanine (Dha), γ-Carboxyglutamic acid (Gla), α-Cyclohexylglycine (Chg), Propargylglycine (Pra), Pyroglutamic acid (Glp), α-tert-Butylglycine (Tle), 4-Benzoylphenylalanine (Bpa), δ-Hydroxylysine (Hyl), 4-Hydroxyproline (Hyp), allo-Isoleucine (alle), Lanthionine (Lan), (1-naphthyl)alanine (1-Nal), (2-naphthyl)alanine (2-Nal), Norleucine (Nle), Norvaline (Nva), Ornithine (Orn), Phenylglycin (Phg), Pipecolic acid (Pip), Sarcosine (Sar), Selenocysteine (Sec), Statine (Sta), □-Thienylalanine (Thi), 1,2,3,4-Tetrahydroisochinoline-3-carboxylic acid (Tic), allo-Threonine (aThr), Thiazolidine-4-carboxylic acid (Thz), γ-Aminobutyric acid (GABA), iso-Cysteine (iso-Cys), Diaminopropionic acid (Dpr), 2,4-Diaminobutyric acid (Dab), 3,4-Diaminobutyric acid (γ,βDab), Biphenylalanine (Bip), Phenylalanine substituted in para-position with -C₁-C₆-alkyl, -halide, -NH₂ or -CO₂H (Phe(4-R) wherein R = -C₁-C₆-alkyl, -halide, -NH₂, or -CO₂H); peptide nucleic acids (PNA, cf. P.E. Nielsen, Acc.Chem.Res. 32, 624-30)
• or their N-alkylated analogues, such as their N-methylated analogues.

Cyclic amino acids may be proteinogenic or non-proteinogenic, such as Pro, Aze, Glp, Hyp, Pip, Tic and Thz.

For further examples and details reference can be made to e.g. J.H. Jones, J. Peptide Sci. 2003, 9, 1-8 which is incorporated herein by reference.

The terms "non-proteinogenic amino acid" and "non-proteinogenic amino acid residue" also encompasses derivatives of proteinogenic amino acids. For example, the sidechain of a proteinogenic amino acid residue may be derivatized thereby rendering the proteinogenic amino acid residue "non-proteinogenic". The same applies to derivatives of the C-terminus and/or the N-terminus of a proteinogenic amino acid residue terminating the amino acid sequence.

For the purpose of the specification a proteinogenic amino acid residue is derived from a proteinogenic amino acid selected from the group consisting of Ala, Arg, Asn, Asp, Cys, Gln, Glu, Gly, His, Ile, Leu, Lys, Met, Phe, Pro, Ser, Thr, Trp, Tyr and Val either in L- or D-configuration; the second chiral center in Thr and Ile may have either R- or S-configuration. Therefore, for example, any posttranslational modification of an amino acid sequence, such as N-alkylation, which might naturally occur, renders the corresponding modified amino acid residue "non-proteinogenic", although in nature said amino acid residue is incorporated in a protein.

C is a metal ion chelator.

For the purpose of this invention, the metal ion chelator C is suitable for complexing of lanthanide and lanthanide like metals. Such chelators are well known in the literature and examples are given in S Liu, DS Edwards, Bifunctional Chelators for Therapeutic Lanthanide Radiopharmaceuticals, Bioconjugate Chemistry 12 (2001) 7 - 34.

In preferred embodiments of compounds according to Formula I, C is selected from the group of:
a) DO3A-maleimide, 10-{2-[(2-{[2-(2,5-dihydro-2,5-dioxo-1*H*-pyrrol-1-yl)ethyl]amino}-2-oxoethyl)amino]-1-methyl-2-oxoethyl}-1 ,4,7,10-tetraazacyclododecane-1,4,7-triacetic acid 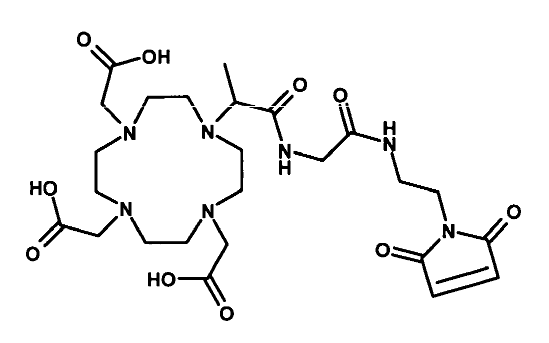
b) DOTA-type ligands according to the general formula given below: 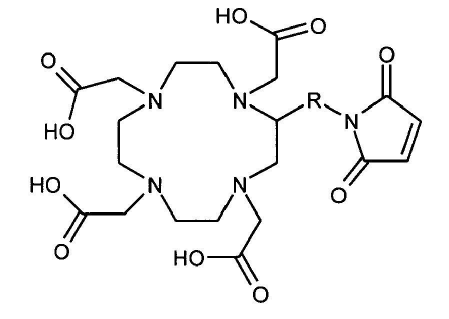 wherein,
   - R: is selected from substituted or un-substituted lower un-branched or branched alkyl, substituted or un-substituted lower un-branched or branched alkene, or a chemical structure comprising at least one amino acid, or an amino acid sequence of two to 20 amino acids.
c) 1,4,7-Tris(2-mercaptoethyl)-1,4,7-triaza-cyclononane-type ligands' 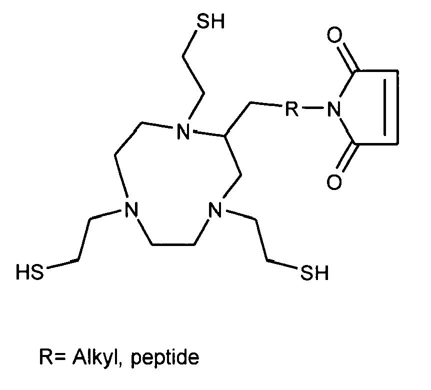 , wherein
   - R: is selected from substituted or un-substituted lower un-branched or branched alkyl, substituted or un-substituted lower un-branched or branched alkene, or a chemical structure comprising at least one amino acid, or an amino acid sequence of two to 20 amino acids.
d) 1,4,7-triazacyclononane-1,4,7 triacetic acid type ligands (NOTA) 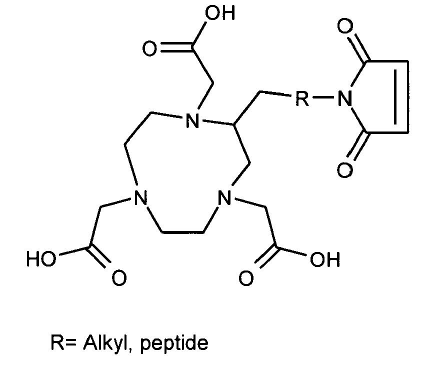 , wherein
   - R: is selected from substituted or un-substituted lower un-branched or branched alkyl, substituted or un-substituted lower un-branched or branched alkene, or a chemical structure comprising at least one amino acid, or an amino acid sequence of two to 20 amino acids.
e) 1,4,7-Tris(3,5-dimethyl-2-hydroxybenzyl)-1,4,7-triaza-cyclononane-type ligands 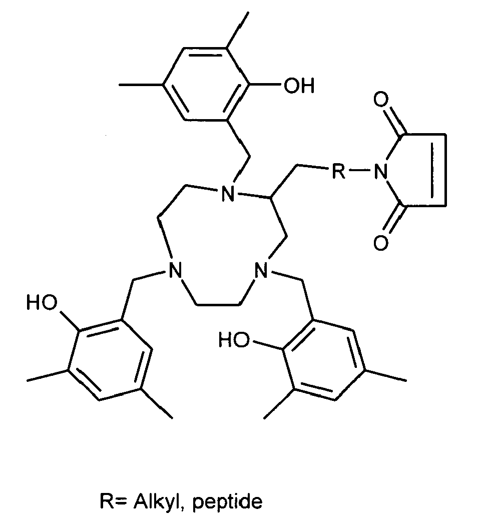 , wherein
   - R: is selected from substituted or un-substituted lower un-branched or branched alkyl, substituted or un-substituted lower un-branched or branched alkene, or a chemical structure comprising at least one amino acid, or an amino acid sequence of two to 20 amino acids.
f) Tripodalligands 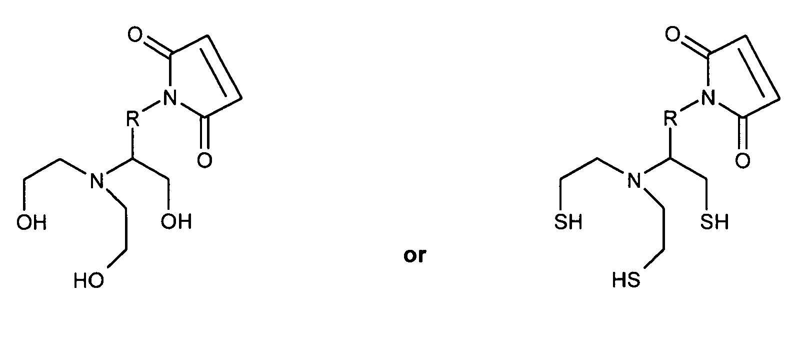 , wherein
   - R: is selected from substituted or un-substituted lower un-branched or branched alkyl, substituted or un-substituted lower un-branched or branched alkene, or a chemical structure comprising at least one amino acid, or an amino acid sequence of two to 20 amino acids.

Unless otherwise specified, when referring to the compounds of Formula I per se, labeled compounds according to Formula I, as well as to any pharmaceutical composition thereof the present invention includes all of the hydrates, solvates, complexes, and prodrugs of the compounds of the invention. Prodrugs are any covalently bonded compounds, which releases the active parent pharmaceutical according to Formula I.

In a further aspect of the invention, compounds according to Formula I and any preferred embodiments are provided that are labeled with a lanthanide or lanthanide like metal ion. Methods and conditions for such labeling reactions are well known to the skilled person (RE Weiner, ML Thakur, Chemistry of Gallium and Indium Radiopharmaceuticals, in MJ Welch, CS Redvanly, Handbook of Radiopharmaceuticals, Wiley (2003) 363 - 399).
A major advantage of the lanthanide and lanthanide like metals is that there is no redox-chemistry involved. The oxidation state of indium, gallium and yttrium is +3 in aqueous solution. According to the HSAB theory In(III), Ga(III) and Y(III) are hard metals and thus prefer hard donor atoms such as oxygen and nitrogen (CJ Anderson, MJ Welch, Radiometal-Labeled Agents (Non-Technetium) for Diagnostic Imaging, Chem. Rev. 99 (1999) 2219 - 2234). Indium, gallium and yttrium share similar ionic radii and a similar coordination chemistry in aqueous solution, which is comparable to the coordination chemistry of lanthanide metals (S Liu, DS Edwards, Bifunctional Chelators for Therapeutic Lanthanide Radiopharmaceuticals, Bioconjugate Chemistry 12 (2001) 7 - 34).

To increase thermodynamic stability and kinetic inertness of a metal complex, polydentate chelators are useful. Lanthanide and lanthanide like metals have the ability to form very stable complexes with a variety of polydentate ligands. Most commonly used ligands for these metal ions are diethylene-triamine pentaacidic acid (DTPA) and 1,4,7,10-tetraazacyclododecane-1,4,7,10-tetraacetic acid (DOTA) derivatives".

Polydentate ligands such as DOTA derivatives with a three dimensional cavity are of particular interest because of their ability to adopt a preorganized conformation in the uncomplexed form.

Preferred isotopes are ¹¹¹In, ⁶⁷Ga, ⁶⁸Ga, ⁸⁶Y, ⁹⁰Y and ¹⁷⁷Lu.

In terms of availability and half-life, ⁶⁸Ga is an imaging isotope with optimal properties, being a potential metallic surrogate for ¹⁸F in PET applications. Since the 1970's ⁶⁸Ge/⁶⁸Ga generator systems have been under evaluation, and thus, over the last two decades ⁶⁸Ga chemistry has been studied (Green MS, Welch MJ, Gallium radiopharmaceutical chemistry, Int. J. Rad. Appl. Instrum B 16 (1989) 435 - 448; Moerlein SM, Welch MJ, The chemistry of gallium and indium as related to radiopharmaceutical production, Int. J. Nucl. Med. Biol. 8 (1981) 277 - 287; Hnatowich DJ, A review of radiopharmaceutical development with short-lived generator-produced radionuclides other than Tc-99m, Int J Appl Radiat Isot 28 (1977) 169-181).

In an aspect of the invention compounds according to Formula I and any preferred embodiments are provided that are labeled with ⁶⁷Ga, ⁶⁸Ga, ⁸⁶Y**,** ⁹⁰Y, ¹⁷⁷Lu, or ¹¹¹In.

In further preferred embodiments compounds of Formula I are labeled with ⁶⁸Ga.

If a chiral center or another form of an isomeric center is present in a compound according to Formula I or labeled compounds of Formula I of the present invention, all forms of such isomer, including enantiomers and diastereoisomers, are intended to be covered herein. Compounds containing a chiral center may be used as racemic mixture or as an enantiomerically enriched mixture or the racemic mixture may be separated using well-known techniques and an individual enantiomer may be used alone. In cases in which compounds have unsaturated carbon-carbon bonds (double bonds), both the cis-isomer and trans-isomers are within the scope of this invention. In cases wherein compounds may exist in tautomeric forms, such as keto-enol tautomers, each tautomeric form is contemplated as being included within this invention whether existing in equilibrium or predominantly in one form.

The invention also provides compositions comprising a compound of Formula I or labeled compounds according to Formula I and a pharmaceutically acceptable carrier or diluent. Pharmaceutically acceptable carrier or diluent may include any and all solvents, dispersion media, antibacterial and antifungal agents, isotonic agents, enzyme inhibitors, transfer ligands such as glucoheptonate, tartrate, citrate, or mannitol, and the like. Such compositions may be formulated as sterile, pyrogen-free, parenterally acceptable aqueous solution which may optionally be supplied in lyophilized form. The compositions of the invention may be provided as components of kits which may include buffers, additional vials, instructions for use, and the like.

In a further aspect the present invention provides a kit comprising a sealed vial containing a predetermined quantity of a compound according to Formula I.

In another aspect of the invention compounds according to Formula I and labeled compounds according to Formula I are provided for use as medicament.

In another aspect of the invention compounds according to Formula I and labeled compounds according to Formula I are provided for use as diagnostic imaging agent, preferably as imaging agent for PET applications.

In a further aspect of the invention, compounds according to Formula I and labeled compounds according to Formula I are provided for the use in manufacturing of a medicament.

In a further aspect of the invention, compounds according to Formula I and labeled compounds according to Formula I are provided for the use in manufacturing of a diagnostic imaging agent.

In a further aspect of the invention, compounds according to Formula I and labeled compounds according to Formula I are provided for the use in manufacturing of a PET imaging agent.

The radioactively labeled compounds according to Formula I provided by the invention may be administered intravenously in any pharmaceutically acceptable carrier, e.g. conventional medium such as an aqueous saline medium, or in blood plasma medium, as a pharmaceutical composition for intravenous injection. Such medium may also contain conventional pharmaceutical materials such as, for example, pharmaceutically acceptable salts to adjust the osmotic pressure, buffers, preservatives and the like. Among the preferred media are normal saline and plasma. Suitable pharmaceutical acceptable carriers are known to the person skilled in the art. In this regard reference can be made to e.g. Remington's Practice of Pharmacy, 11^{th} ed.

The concentration of the compound according to Formula I and the pharmaceutically acceptable carrier, for example, in an aqueous medium, varies with the particular field of use. A sufficient amount is present in the pharmaceutically acceptable carrier when satisfactory visualization of the imaging target (e.g. a tumor) is achievable.

In accordance with the invention, the radiolabeled compounds according to Formula I either as a neutral complex or as a salt with a pharmaceutically acceptable counter-ion are administered in a single unit injectable dose. Any of the common carriers known to those with skill in the art, such as sterile saline solution or plasma, can be utilized after radiolabeling for preparing the injectable solution to diagnostically image various organs, tumors and the like in accordance with the invention. Generally, the unit dose to be administered for a diagnostic agent has a radioactivity of about 0.1 mCi to about 100 mCi, preferably 1 mCi to 20 mCi. For a radiotherapeutic agent, the radioactivity of the therapeutic unit dose is about 10 mCi to 700 mCi, preferably 50 mCi to 400 mCi. The solution to be injected at unit dosage is from about 0.01 ml to about 30 ml. For diagnostic purposes after intravenous administration, imaging of the organ or tumor in vivo can take place in a matter of a few minutes. However, imaging takes place, if desired, in hours or even longer, after injecting into patients. In most instances, a sufficient amount of the administered dose will accumulate in the area to be imaged within about 0.1 of an hour to permit the taking of scintigraphic images. Any conventional method of scintigraphic imaging for diagnostic purposes can be utilized in accordance with this invention.

### BRIEF DESCRIPTION OF THE FIGURES

- Figure 1:: Binding Assay of ¹¹¹In-TTA1-MAG₂-DO3A (**15A**) to tenascin-C. Increasing amounts of TN-C (1.25 x 10⁻⁷ M to 6.35 x 10⁻¹² M) are plotted against the radioactivity that was bound to the nitrocellulose filter. From the curve a K_{D} of 1 nM was calculated.
- Figure 2:: ⁶⁸Ga-TTA1-MAG₂-DO3A microPET imaging in a U251 tumor bearing nude mouse, as observed at 1 h p.i. An uptake of the radioactive tracer was found in the tumor. Furthermore, an uptake of the radioactivity was observed in the kidneys which were cleared through the bladder.

### EXAMPLES

### Example 1: Synthesis of Aptamer-D03A conjugates

### a) Synthesis of 10-[2-({2-[(2-{2,5-dioxo-3-[(2-oxo-2-{[2-oxo-2-({2-oxo-2-[(TTA1)-ylamino]ethyl}amino)ethyl]amino}ethyl)sulfanyl]pyrrolidine-1-yl}ethyl)amino]-2-oxoethyl}amino)-1-methyl-2-oxoethyl]-1,4,7,10-tetraazacyclododecane-1,4,7 triacidic acid

### TTA1-MAG₂-DO3A

1.2 mg TTA1-MAG₂ dissolved in 30 µl of a 0.2 M phosphate buffer (pH 7.4) was incubated with 1 mg (1.5 µmol) DO3A-maleimid at 37 °C for one hour. Excess of DO3A-maleimide was removed by spin dialysis at 13 000 rpm using a 10 kDa cutoff filter at 13 000 rpm. The product on the filter was washed three times with 100 µl water and filtered at 13 000 rpm for 20 minutes. The sample was stored at - 70 °C.

### Analysis

Mass (ESI⁻): Calcd [M]: 14055; found [m/z]: 14094 (M + K⁺)
The reaction scheme of the synthesis of 10-[2-({2-[(2-{2,5-dioxo-3-[(2-oxo-2-{[2-oxo-2-({2-oxo-2-[(TTA1)-ylamino]ethyl}amino)ethyl]amino}ethyl)sulfanyl]pyrrolidin-1-yl}ethyl)amino]-2-oxoethyl}amino)-1-methyl-2-oxoethyl)-1,4,7,10-tetraazacyclododecane-1,4,7-triacetic acid (TTA1-MAG₂-DO3A) (15) are given below:.

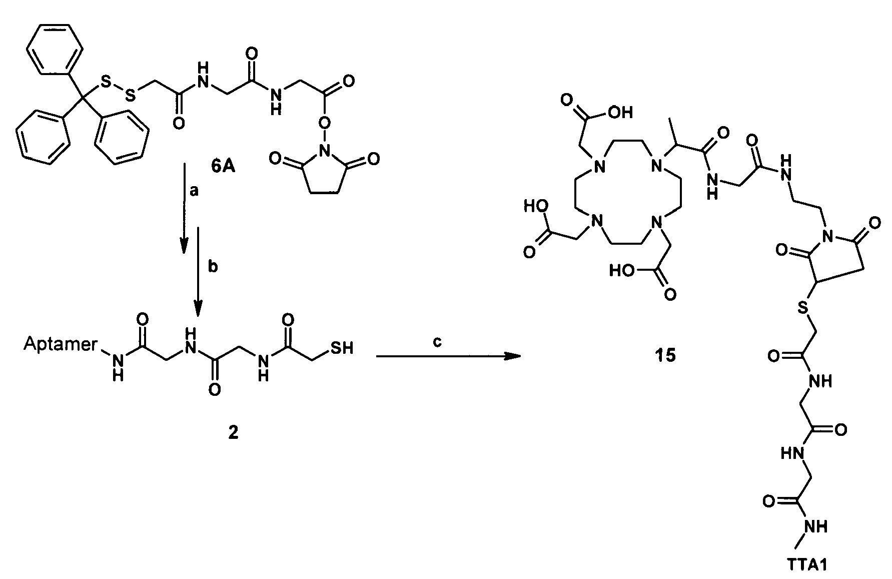

### Synthesis of TTA1-MAG₂-DO3A (15)

### a) TTAI-MAG2 (2), borate buffer, rt, b) TCEP, acetate buffer, rt, c) D03A-maleimide (6a), borate buffer, rt

### b) Synthesis of 10-(2-{[2-({2-[3-((4-imino-4-[(TTAI)-ylamino]butyl)sulfanyl)-2,5-dioxopyrrolidine-1-yl]ethyl}amino)-2-oxoethyl]amino}-1-methyl-2-oxoethyl)- 1,4,7,10-tetraazacyclododecane-1,4,7-triacetic acid

### TTA7-MBI-DO3A

1.2 mg (100 nmol) TTA7-NH₂ dissolved in 15 µl borate buffer (0.2 M, pH 8) and 0.28 mg (2 µmol) 2-iminothiolane dissolved in 5 µl borate buffer (0.2 M, pH 8) were incubated at 0 °C for one hour. Afterwards, excess of 2-iminothiolane was removed by spin filtration using a 10 kDa cutoff filter at 13 000 rpm. The product on the filter was washed three times with 100 µl phosphate buffer (0.1 M, pH 7.4) and filtered at 13 000 rpm for 20 minutes.

Subsequently, the product present in 25 µl phosphate buffer (0.1M, pH 7.4) was reacted with 1 mg (1.5 µmol) DO3A-maleimide (8) at 37 °C for two hours. Excess of DO3A-maleimide was removed by spin dialysis using a 10 kDa cutoff filter at 13 000 rpm. The product on the filter was washed three times with 100 µl water and filtered at 13 000 rpm for 20 minutes. The sample was stored at - 70 °C.

The reaction scheme of the synthesis of 10-(2-{[2-[3-({4-imino-4-[(TTA7)-ylamino]butyl}sulfanyl)-2,5-dioxopyrrolidin-1-yl]ethyl}amino)-2-oxoethyl]amino}-1-methyl-2-oxoethyl)-1,4,7,10-tetraazacyclododecane-1,4,7-triacetic acid (TTA7-MBI-DO3A) (16) is given below.

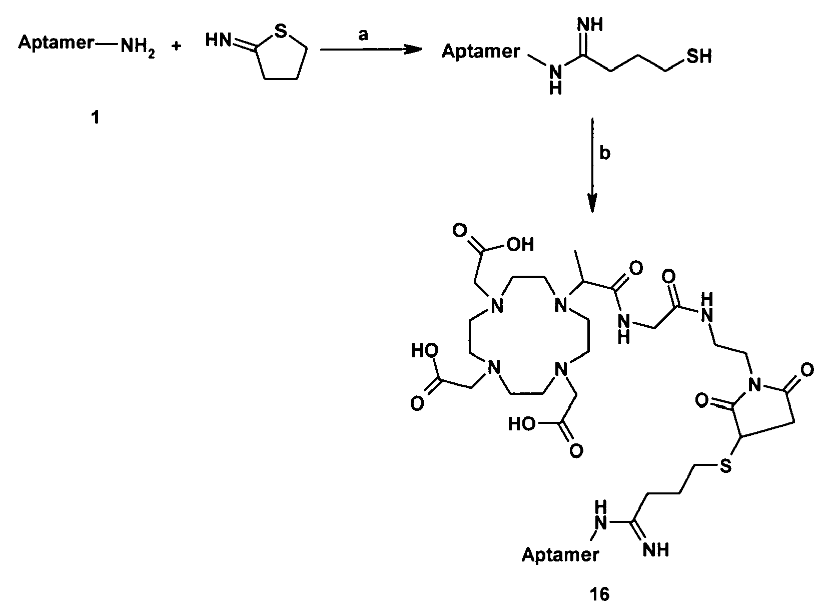

### Synthesis of TTA7-MBI-DO3A (16) a) phosphate buffer, rt, b) D03A-maleimide (8), phosphate buffer, rt

### Example 2: ⁶⁸Ga(III) labeling of aptamer-D03A conjugates

### -General Remarks-

Labeling requirements for lanthanide like metals differ from ^{99m}Tc and ¹⁸⁸Re labeling conditions. The buffering system needed to be changed from a sodium phosphate buffer to an ammonium acetate buffer in order to prevent the formation of insoluble metal phosphate compounds. In fact, Liu et al speculates that the formation of metal phosphate compounds might be the reason for the high affinity of lanthanide like metal ions to localize in the bone *in vivo.* Acetate is known to be a suitable auxiliary ligand for further ligand exchange reactions with lanthanide like isotopes. A precipitation of metal hydroxide usually formed at a pH > 3 can thus be prevented. This was important as the pH had to be raised to 5.5 and higher for the labeling reaction of aptamer conjugates, due to decomposition of the aptamer at pH < 5.5. The lanthanide like metal acetate auxiliary ligand complex was prone to subsequent rapid conversion in the presence of polydentate N,O-chelating ligands such as DO3A and MX-DTPA.

### a) ⁶⁸Ga labeling of TTA1-MAG₂-DO3A

200 µg TTA1-MAG₂-DO3A (15 nmol) were dissolved in 400 µl ammonium acetate buffer (pH 5.5, 0.5 M) solution. 100 µl (100 - 300 MBq) [⁶⁸Ga]GaCl₃ eluate were added and subsequently the reaction mixture was incubated at 100 °C for twenty minutes. The product was purified by spin filtration at 13 000 rpm employing a 10 k Da cutoff filter.

The purity of the product determined by PAGE and HPLC (table 9.6) was > 90 %.

### b) ¹¹¹In labeling of TTA7-MBI-DO3A

¹¹¹In labeling of TTA7-MBI-DO3A was accomplished in analogy to the ¹¹¹In labeling of TTA1-MAG₂-DO3A. Product formation was achieved at a pH of 5.5 using an ammonium acetate buffer. The reaction was proceeded at 100 °C for 20 minutes. Product yield was up to 70 % after purification by spin filtration. Purity of the product exceeded always 95 %. Compound identity was proven by HPLC and PAGE (data not shown). The results were in accordance with the data obtained for ¹¹¹In-TTA1-MAG₂-DO3A.

### Example 3: Binding affinity

### a) Binding affinity of compounds according to Formula I to human tenascin-C

A competition filter binding assay was carried out in order to determine binding constants (EC₅₀). Samples of 2 nM human TN-C were incubated with 1 nM ^{99m}Tc-TTA1-MAG₂ in TBSMC buffer (20 mM Tris, pH 7.4; 137 mM NaCl; 1 mM CaCl₂; 1 mM MgCl₂) and the binding competed with varying concentrations of unlabeled aptamer (see pipetting scheme in figure 9.1). Incubation was carried out at 37 °C for 15 min. Subsequently, the solutions were pipetted on a Minifold Device equipped with a supported nitrocellulose membrane and Whatman filter paper and were washed with buffer using vacuum. Residual radioactivity on the filter due to tenascin-C bound labeled aptamer was quantified using a Phosphorimager. EC₅₀ values were calculated using GraphPadPrism 3.02 (San Diego, CA, USA) plotting one-side competition non-linear regression curves.

**Table 2: Binding affinity of preferred aptamers against Tenascin-C**

| **Aptamer** | **EC50 in nM** |
|---|---|
| TTA1 | 6 |
| TTA2 | 31 |
| TTA3 | 19 |
| TTA4 | 20 |
| TTA5 | 39 |
| TTA6 | 14 |
| TTA7 | 2 |
| TTA8 | 14 |

### b) Binding affinity of ¹¹¹In-TTA1-MAG₂-DO3A

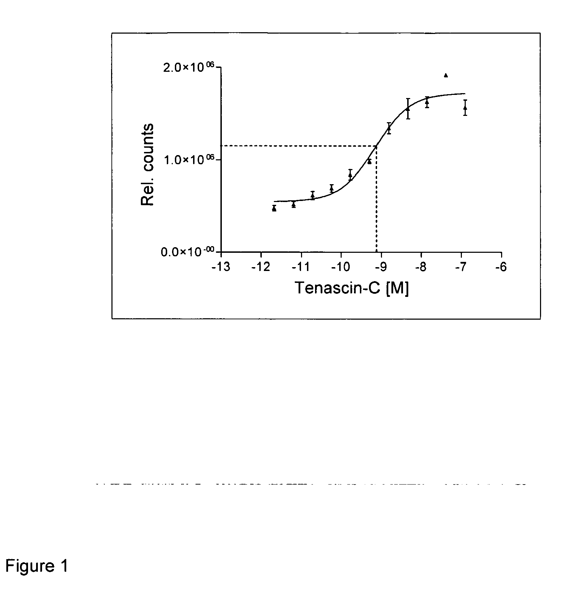

As an example from the group of the aptamer conjugates labeled with lanthanide like metals ¹¹¹In-TTA1-MAG₂-DO3A was investigated for its binding affinity for human tenascin-C. The dissociation constant K_{D} was calculated directly by incubating a given concentration of the radiolabeled compound ¹¹¹In-TTA1-MAG₂-DO3A with varying amounts of tenascin-C (1.25 x 10⁻⁷ M to 6.35 x 10⁻¹² M). The K_{D} for ¹¹¹In-TTA1-MAG₂-DO3A was determined to be 1 nM.
An illustration of the binding curve is depicted in Figure 1.

### Example 4: Biodistribution studies

### a) Biodistribution studies of ⁶⁸Ga-TTA 1-MAG₂-DO3A

⁶⁸Ga-TTA1-MAG₂-DO3A was studied in biodistribution experiments using U251 tumor bearing nude NMRI mice. Due to the short half-life of ⁶⁸Ga, only early time points were chosen for this study.
The tumor uptake of ⁶⁸Ga-TTA1-MAG₂-DO3A was detected to be 2.0 % ID/g at 1 h post injection. At the same time point, only 0.4 % ID/g were observed in the blood. Kidney and liver uptake at 1 h p.i. were found to be lower compared to the ¹¹¹In labeled compounds. Clearance of ⁶⁸Ga-TTA1-MAG₂-DO3A was primarily via the urine (71.7 % ID at 1 h p.i.), whereas only 0.9 % ID/g were found in the intestine at this time point.

**Table 3: Clearance of ⁶⁸Ga-TTA1-MAG₂-DO3A over time**

| | **⁶⁸Ga- TTA1-MAG₂-DO3A** | |
|---|---|---|
| | 5 min p.i. | 1 h p.i. |
| Intestine (% ID/g) | 1.9±0.2 | 0.9±0.6 |
| Urine (% ID) | 40.9±5.6 | 71.7±6.4 |

**Table 4: Tumor to organ ratios for tumor to blood (T/B), tumor to kidney (T/K), and tumor to liver (T/L)**

| | **T** **(%ID/g)** | **T/B** **(%ID/g)** | **T/K** **(%ID/g)** | **T/L** **(%ID/g)** |
|---|---|---|---|---|
| ⁶⁸Ga-TTA1-DO3A | 2.0 | 5.1 | 0.2 | 0.7 |

### b) Comparison of Biodistribution of ^{99m}Tc-TTA1-MAG₂ vs. ⁶⁸Ga-TTA1-MAG₂-DO3A

The biodistributions of radiolabeled aptamers were investigated in nude mice bearing subcutaneous human U251 glioblastoma. Expression of human TN-C in the U 251 xenograft has previously been confirmed using an ¹²⁵I-anti-TN-C antibody. Female nude mice (NMRI-Foxn1^{nu}, Taconic), of approximately 22 g body weight were inoculated with 2.2 x 10⁶ U 251 human glioblastoma cells (ATCC) in 100 µl PBS subcutaneously in the right hind flank. After 3 - 4 weeks, mice with palpable tumors (approximately 100 mg) were injected with 74-111 KBq radiolabeled aptamer conjugate into the tail vein. After 0.25, 1, 5 and 24 h post injection (p.i.), three mice were sacrificed per time-point and selected organs counted for radioactivity in a gamma-counter (Compugamma LKB Wallac). %ID/g (% injected dose per gram tissue) and %ID (% injected dose) were calculated for organs, urine and feces.

Table 5 depicts the results from the biodistribution study in tumor bearing mice in comparison to the data for ^{99m}Tc-TTA1 -MAG₂.

**Table 5: Comparison of biodistribution of ⁶⁸Ga-TTA1-MAG₂-DO3A vs. ^{99m}Tc-TTA1-MAG₂**

| | Tumor (%ID/g) | Blood (%ID/g) | Kidney (%ID/g) | Liver (%ID/g) | Urine (%ID) | Intestine (%ID) |
|---|---|---|---|---|---|---|
| ⁶⁸Ga-TTA1-MAG₂-DO3A | 1.95±0.55 | 0.38±0.07 | 8.8±3.5 | 2.79±0.89 | 71.7±6.4 | 0.9±0.6 |
| ^{99m}Tc-TTA1-MAG₂ | 0.73±0.34 | 0.28±0.04 | 0.62±0.06 | 0.76±0.43 | 38.4±3.3 | 14.1 ±2.0 |

Table 6: Selected data from the organ distribution study of ⁶⁸Ga-TTA1-MAG₂-DO3A and ^{99m}Tc-TTA1-MAG₂ in U251 tumor bearing nude NMRI mice (n = 3 per time point, ID = injected dose). Bars represent the percentage of injected dose per gram (% ID/g) of tissue at 1 h post injection in tumor, blood, kidney and liver. The table (b) depicts the % ID/g in the intestine and the % ID values that were found in the urine at 1 h post injection.

| | Tumor/Blood | Tumor/Kidney | Tumor/Liver |
|---|---|---|---|
| ⁶⁸Ga-TTA41-MAG₂-DO3A | 5.13 | 0.22 | 0.70 |
| ^{99m}Tc-TTA1-MAG₂ | 2.61 | 1.18 | 0.96 |

### Example 5: In vivo imaging of ⁶⁸Ga-TTA1-MAG₂-DO3A

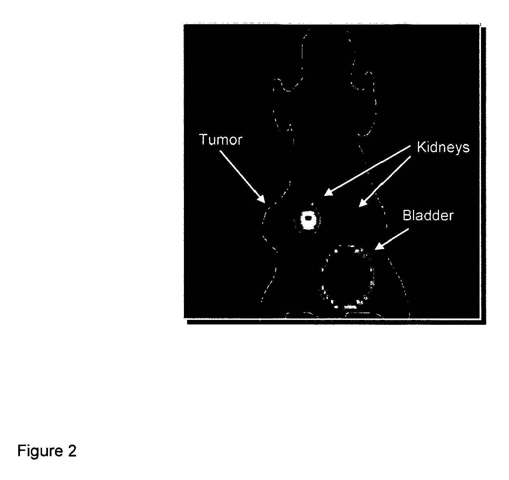

40 MBq of the labeled compound were injected into U251 tumor bearing nude mice and a dynamic PET imaging study was performed. Due to the short half-life of ⁶⁸Ga, imaging was only performed until 1 h post injection, see Figure 2.
A high tumor to blood ratio was expected based on the biodistribution results. The tumor was well visible at 1 h post injection. The kidneys were the organ with the highest tracer uptake. Furthermore, the bladder was very prominent.. No liver or intestinal uptake was observed at the 1 h time point.

## Claims

1. Compound according to Formula I:
A-B-L-C (I)
, wherein
A stands for an aptamer,
B is absent or stands for a bridging structure,
L is a linker, and
C is a metal ion chelator.

2. A compound according to claim 1, wherein the A is selected from Tenascin-C binding aptamers.

3. A compound according to claims 1 and 2, wherein A is an aptamer having a binding affinity towards Tenascin-C of 1mM or less.

4. A compound according to claims 1 to 3, wherein A comprises from 10 to 50 nucleotides.

5. A compound according to claims 1 to 4, wherein B is hexyl amine.

6. A compound according to claims 1 to 5, wherein C is selected from the group of:
a) D03A-maleimide, 10-{2-[(2-{[2-(2,5-dihydro-2,5-dioxo-1*H*-pyrrol-1-yl)ethyl]amino}-2-oxoethyl)amino]-1-methyl-2-oxoethyl}-1,4,7,10-tetraazacyclododecane-1,4,7-triacetic acid 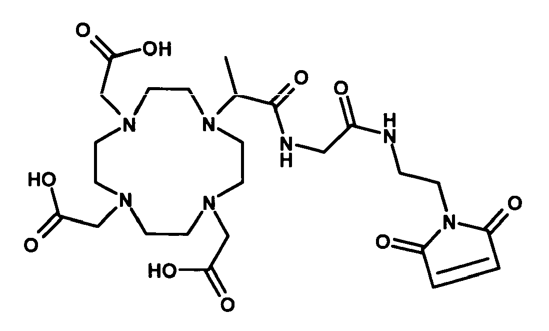
b) DOTA-type ligands according to the general formula given below: 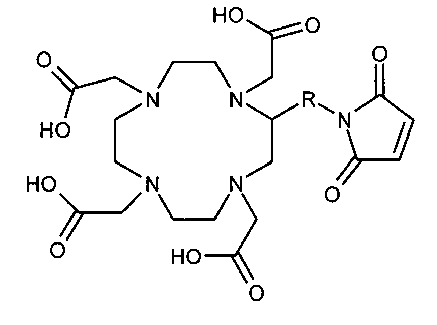 , wherein
R is selected from substituted or un-substituted lower un-branched or branched alkyl, substituted or un-substituted lower un-branched or branched alkene, or a chemical structure comprising at least one amino acid, or an amino acid sequence of two to 20 amino acids.
c) 1,4,7-Tris(2-mercaptoethyl)-1,4,7-triaza-cyclononane-type ligands 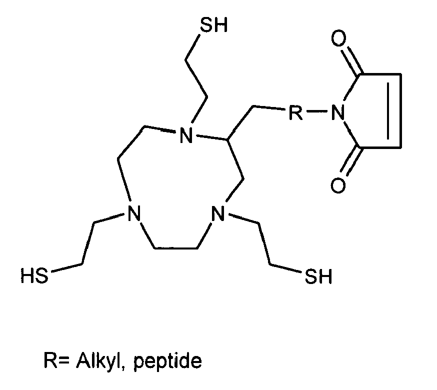 , wherein
R is selected from substituted or un-substituted lower un-branched or branched alkyl, substituted or un-substituted lower un-branched or branched alkene, or a chemical structure comprising at least one amino acid, or an amino acid sequence of two to 20 amino acids.
d) 1,4,7-triazacyclononane-1,4,7 triacetic acid type ligands (NOTA) 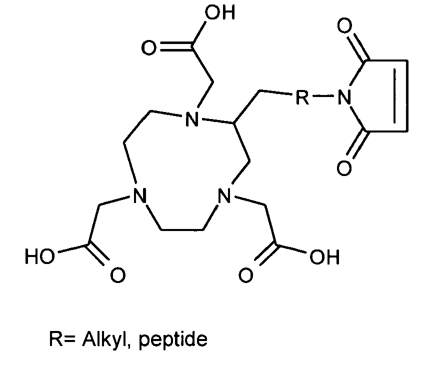 , wherein
R is selected from substituted or un-substituted lower un-branched or branched alkyl, substituted or un-substituted lower un-branched or branched alkene, or a chemical structure comprising at least one amino acid, or an amino acid sequence of two to 20 amino acids.
e) 1,4,7-Tris(3,5-dimethyl-2-hydroxybenzyl)-1,4,7-triaza-cyclononane-type ligands 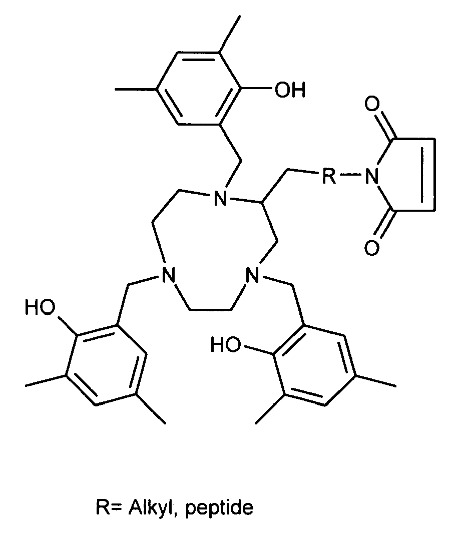 , wherein
R is selected from substituted or un-substituted lower un-branched or branched alkyl, substituted or un-substituted lower un-branched or branched alkene, or a chemical structure comprising at least one amino acid, or an amino acid sequence of two to 20 amino acids.
f) Tripodalligands 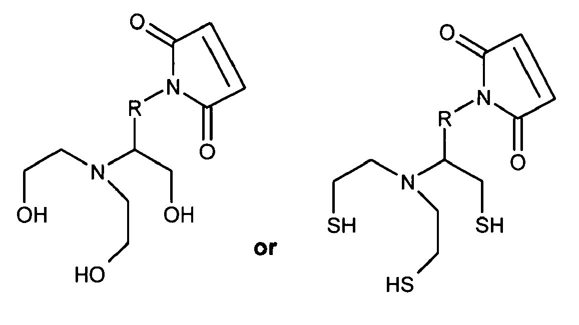 , wherein
R is selected from substituted or un-substituted lower un-branched or branched alkyl, substituted or un-substituted lower un-branched or branched alkene, or a chemical structure comprising at least one amino acid, or an amino acid sequence of two to 20 amino acids.

7. A compound according to claims 1 to 6, wherein L is a chemical structure carrying at least one free mercapto-group, wherein said chemical structure comprises an amino acid sequence of 2 to 10 amino acids, MAG₂, mercaptobutyrimidyl, mercapto-substituted C₁-C₄ alkyl, mercapto-substituted C₁-C₄ alkene, or mercapto-substituted C₁-C₄ aldehyde.

8. A compound according to claims 1 to 7, wherein L is MAG₂.

9. A compound according to claims 1 to 8, wherein A - B is selected from the group of compounds:
| | |
|---|---|
| **TTA1:** | 5'-NH2-C6-O-PO2-O-GGG AGG ACG-(CH2CH2O)6-CGU CGC CGU AAU GGA UGU UUU GCU CCC UG-3'-3'-T , |
| **TTA2:** | 5'-NH2-C6-O-PO2-O-mGmGmG mAmGG mAfCmG-(CH2CH2O)6-fCGfU fCGfC fCGfU mAmAfU mGmGmA fUmGfU fUfUfU mGfCfU fCfCfC UmG-3'-3'-T , |
| **TTA3:** | 5'-NH2-C6-O-PO2-O-mGmGmG mAmGG mAfCmG-(CH2CH2O)6-fCGfU fCGfC fCGfU mAmAfU mGmGmA fUmGfU fUfUfU mGfCfU fCfCfC UG-3'-3'-T , |
| **TTA4:** | 5'-mGmGmG mAmGG mAfCmG-(CH2CH2O)6-fCGfU fCGfC fCGfU mAmAfU mGmGmA fUmGfU fUfUfU mGfCfU fC(C6-NH2)CfC-3'-3'-T , |
| **TTA5:** | 5'-(aminomodifier C2 dT)-mA mGmGmG mAmGG mAfCmG-(CH2CH2O)6-fCGfU fCGfC fCGfU mAmAfU mGmGmA fUmGfU fUfUfU mGfCfU fCfCfC fUmG-3'-3'-T , |
| **TTA6:** | 5'-NH2-C6-O-SPO-O-mGmGmG mAmGG mAfCmG-(CH2CH2O)6-fCGfU fCGfC fCGfU mAmAfU mGmGmA fUmGfU fUfUfU mGfCfU fCfCfC fUmG-3'-3'-T , |
| **TTA7:** | 5'-LGLGLG LALGmG mAfCrG-(CH2OCH2)6-fCGfU fCGfC fCGfU mAmAfU mGmGmA fUmGfU fUfUfU mGLCLU LCLCLC-(CH2)6NH2-3', and |
| **TTA8:** | 5'-mGmGmG mAmGmG mAfCrG-(CH2OCH2)6-fCGfU fCGfC fCGfU mAmAfU mGmGmA fUmGfU fUfUfU mGmCmU mCmCmC-(CH2)6NH2-3' |

10. A compound according to claims 1 to 9, wherein the compound is labeled with a lanthanide or lanthanide like metal ion.

11. A compound according to claims 1 to 10, wherein the compound is labeled with ⁶⁷Ga, ⁶⁸Ga, ⁸⁶Y, ⁹⁰Y, ¹⁷⁷Lu, or ¹¹¹In.

12. A compound according to claims 1 to 11, wherein the compound is labeled with ⁶⁸Ga.

13. A composition comprising a compound according to claims 1 to 12 and a pharmaceutically acceptable carrier or diluent.

14. A kit comprising a sealed vial containing a predetermined quantity of a compound according to claims 1 to 12.

15. A compound according to claims 1 to 12 for use as a medicament.

16. A compound according to claims 1 to 12 for use as a diagnostic imaging agent, preferably as imaging agent for PET.

17. Use of a compound according to claims 1 to 12 for the manufacture of a medicament.

18. Use of a compound according to claims 1 to 12 for the manufacture of a diagnostic imaging agent, preferably a PET agent.

19. Use of a compound according to claims 1 to 12 for the manufacture of a diagnostic imaging agent, preferably a PET agent, for imaging tumors.
